# EUROPEAN PATENT SPECIFICATION

(11) **EP 3 375 786 B1**
(45) Date of publication and mention of the grant of the patent: **09.12.2026**
(21) Application number: 18169031.4
(22) Date of filing: 23.12.2014
(51) Int. Cl.: C07H 1/08, C07H 3/06, A61K 31/702, C12P 19/14, A23L 5/00, A23L 29/30, A23L 33/21, A23L 33/00

(54) **PROCESS FOR EFFICIENT PURIFICATION OF NEUTRAL HUMAN MILK OLIGOSACCHARIDES (HMOS) FROM MICROBIAL FERMENTATION**
VERFAHREN ZUR EFFIZIENTEN REINIGUNG VON NEUTRALEN HUMANEN MILCH-OLIGOSACCHARIDEN (HMOS) AUS MIKROBIELLER FERMENTATION
PROCÉDÉ PERMETTANT DE PURIFIER EFFICACEMENT DES OLIGOSACCHARIDES DU LAIT HUMAIN NEUTRE (HMO) À PARTIR DE LA FERMENTATION MICROBIENNE

(30) Priority: 20.01.2014 EP 14151737
(43) Date of publication of application: 19.09.2018
(62) Document number(s) of the earlier application(s) in accordance with Art. 76 EPC: 14827224.8 / 3 131 912
(73) Proprietor: Chr. Hansen HMO GmbH, 53619 Rheinbreitbach (DE)
(72) Inventor: Jennewein, Stefan, 53604 Bad Honnef (DE)
(74) Representative: NVS EPO Representatives

(56) References cited:
- EP-A1- 2 479 263
- EP-A1- 2 857 410
- WO-A1-2013/025104
- WO-A1-2013/185780
- WO-A2-2012/092160
- WO-A2-2012/112777
- ALBERMANN C ET AL: "Synthesis of the milk oligosaccharide 2'-fucosyllactose using recombinant bacterial enzymes", CARBOHYDRATE RESEARCH, PERGAMON, GB, vol. 334, no. 2, 23 August 2001 (2001-08-23), pages 97 - 103, XP004298614, ISSN: 0008-6215, DOI: 10.1016/S0008-6215(01)00177-X
- WATARU SUMIYOSHI ET AL: "Determination of each neutral oligosaccharide in the milk of Japanese women during the course of lactation", BRITISH JOURNAL OF NUTRITION, vol. 89, no. 01, 1 January 2003 (2003-01-01), pages 61 - 69, XP055199354, ISSN: 0007-1145, DOI: 10.1079/BJN2002746

## Description

The present application discloses a simple process for the purification of neutral human milk oligosaccharides (HMOs) produced by microbial fermentation. The process uses a combination of a cationic ion exchanger treatment, an anionic ion exchanger treatment, and a nanofiltration and/or electrodialysis step, which allows efficient purification of large quantities of neutral HMOs at high purity. Contrary to the purification currently used in fermentative production of neutral HMOs, the presented process allows the provision of HMOs without the need of a chromatographic separation. The so purified HMOs may be obtained in solid form by spray drying, as crystalline material or as sterile filtered concentrate. The provided HMOs are free of proteins and recombinant material originating from the used recombinant microbial strains and thus very well-suited for use in food, medical food and feed (e.g. pet food) applications.

Human milk represents a complex mixture of carbohydrates, fats, proteins, vitamins, minerals and trace elements. The by far most predominant fraction is represented by carbohydrates, which can be further divided into lactose and more complex oligosaccharides. Whereas lactose is used as an energy source, the complex oligosaccharides are not metabolized by the infant. The fraction of complex oligosaccharides accounts for up to 1/10 of the total carbohydrate fraction and consists of probably more than 150 different oligosaccharides. The occurrence and concentration of these complex oligosaccharides are specific to humans and thus cannot be found in large quantities in the milk of other mammals, like for example domesticated dairy animals.

The existence of these complex oligosaccharides in human milk is known already for a long time and the physiological functions of these oligosaccharides were subject to medicinal research for many decades (Gura, T. (2014) Nature's first functional food. Science 345(6198) 747-749). For some of the more abundant human milk oligosaccharides, specific functions have already been identified (Bode, L. (2012) Human milk oligosaccharides: every baby needs a sugar mama. Glycobiology 22(9), 1147-1162.; Bode L, Jantscher-Krenn E (2012) Structure-function relationships of human milk oligosaccharides. Adv Nutr 3(3) 383S-391S; Morrow AL, Ruiz-Palacios GM, Altaye M, Jiang X, Guerrero ML, Meinzen-Derr JK, Farkas T, Chaturvedi P, Pickering LK, Newburg DS (2004) Human milk oligosaccharides are associated with protection against diarrhea in breast-fed infants. J Pediatr 145(3) 297-303).

The limited supply and difficulties of obtaining pure fractions of individual human milk oligosaccharides lead to the development of chemical routes to some of these complex molecules. However, synthesis of human milk oligosaccharides by chemical synthesis, enzymatic synthesis or fermentation proofed to be challenging. At least large-scale quantities as well as qualities sufficient for food applications cannot be provided until today. In this regard, particularly chemical synthetic routes to human milk oligosaccharides (e.g. 2'-fucosyllactose; see WO 2010/115935 A1) involve several noxious chemicals, which impose the risk to contaminate the final product.

Due to the challenges involved in the chemical synthesis of human milk oligosaccharides, several enzymatic methods and fermentative approaches were developed (Miyazaki et al., (2010) Methods in Enzymol. 480, 511-524; Murata et al., (1999) Glycoconj. J. 16, 189-195; Baumgartner, F. et al., (2013) Microb. Cell Fact. 12, 40; Lee et al., (2012) Microb. Cell Fact. 11, 48; US 7,521,212 B1 or Albermann et al., (2001) Carbohydr. Res. 334(2) p 97-103). However, these methods - yield complex mixtures of oligosaccharides i.e. the desired product is contaminated with starting material such as lactose, biosynthetic intermediates and substrates such as individual monosaccharides and polypeptides etc.

Processes in the state of the art for purifying individual oligosaccharide products from these complex mixtures are technically complex and also uneconomical for food applications. For the purification of the disaccharides lactose or sucrose from complex mixtures such as whey or molasses, industrial scale processes have been developed which involve multiple crystallizations. The disadvantage of said methods is that they are elaborate and only lead to low yields.

For the purification of complex oligosaccharides from microbial fermentation, such as certain human milk oligosaccharides, gel-filtration chromatography is the method of choice until now. The disadvantage of gel-filtration chromatography is that it cannot be efficiently scaled up and it is unsuitable for continuous operation. Thus, gel-filtration chromatography is not economical and renders it impossible to provide certain human milk oligosaccharides - like 2'-fucosyllactose or lacto-N-tetraose - in reasonably amounts and quality to use them in human food or other applications such as animal food (e.g. pet food). The application as animal feed or pet food is interesting on the basis that also other mammals contain the same or similar neutral complex oligosaccharides in their milk as humans (e.g. 2'-fucosyllactose is also found in the milk of dogs, pigs, chimpanzee) (Castanys-Munzo, E., Martin, J.M & Prieto, P.A. (2013) 2'-fucosyllactose: an abundant, genetically determined soluble glycan present in human milk. Nutr. Rev. 71(12) 773-789).

Another problem is presented by the use of recombinant strains (recombinant bacterial or yeast strains) in the microbial fermentation, resulting in the contamination of the fermentation product with recombinant material. However, contamination with recombinant DNA or proteins is not acceptable by regulators and consumers today. Detection limits in particular for recombinant DNA molecules are very low. In case qPCR based detection is used, which is currently regarded as the gold standard for detection, even as little a single DNA molecules can be detected. Proteins in addition pose the risk of allergic reactions and should therefore be efficiently removed from the desired oligosaccharide product.

Electrodialysis (ED) represents a technique combining dialysis and electrolysis and can be used for the separation or concentration of ions in solutions based on their selective electromigration through semipermeable membranes. First industrial applications of electrodialysis dated back into the early 1960 with the demineralization of cheese whey for the use in infant formula. Further developed applications of electrodialysis include the adjustment of pH of beverages such as wines, grape must, apple juice and orange juice.

The desalination for brackish water for the production of drinking water and the demineralization of milk whey for infant food production represent the largest area of application, today.

The basic electrodialysis principle consists of an electrolytic cell composed of a pair of electrodes submerged into an electrolyte for conduction of ions connected to a direct current generator. The electrode connected to the positive pole of the direct current generator is the anode, and the electrode connected to the negative pole is called cathode. The electrolyte solution then supports the current flow, which results from the movement of negative and positive charge ions towards the anode and cathode respectively. The membranes employed in the electrodialysis are essentially sheets of porous ionexchange resins, owing negative or positive charge groups and therefore addressed as cationic or anionic membrane, respectively. The ion exchanger membranes are usually made of polystyrene carrying a suitable functional group (such as sulfonic acid or a quaternary ammonium group for cationic or anionic membranes, respectively) cross-linked with divinylbenzene. As electrolyte, sodium chloride, or sodium acetate, sodium propionate etc. can be employed. The electodialysis stack is then assembled in such a way that the anionic and cationic membranes are parallel as in a filter press between two electrode blocks that the stream undergoing ion depletion is well separated from the stream undergoing ion enrichment (the two solutions are also referred to as diluate (undergoing ion depletion) and concentrate (undergoing ion enrichment). The heart of electrodialysis process is the membrane stack, which consists of several anion and cation-exchange membranes separated by spacers, and installed between two electrodes. By applying a direct electric current, anions and cations will migrate across the membranes towards the electrodes generating a diluate (desalted) and a concentrate stream.

Generally, the pore size of the employed membranes is rather small in order to prevent diffusion of the product from the diluate into the concentrate stream, driven by the often high concentration differences between the two streams. After separation from biomass, proteins and in particular recombinant DNA molecules (in the size of entire genomes) have to be removed quantitatively from the desired product. If at all possible the electrodialysis of such large molecules (in comparison to the molecular size of HMOs) would be rather lengthy and surely accompanied with significant losses of the desired product from the diluate into the concentrate.

Diafiltration is a process that involves the addition of fresh water to a solution in order to "wash out" or remove membrane permeable components. Thus, diafiltration can be used to separate components on the basis of their molecular size by using appropriate membranes, wherein one or more species are efficiently retained and other species are membrane permeable. In particular, diafiltration by using a nanofiltration membrane is effective for the separation of low molecular compounds from salts. In general, nanofiltration membranes possess a molecular weight cut-off in the range of 150 to 300 Daltons. Today, nanofiltration (NF) is widely used in the dairy industry for the concentration and demineralization of whey. Nanofiltration has already been employed for the enrichment of a human milk oligosaccharide fraction form human milk. In this approach, nanofiltration has been used in combination with enzymatic degradation of lactose to separate the HMO fraction from the milk lactose (Sarney D.B, Hale, C., Frankel, G & Vulfson, E.N. (2000) A novel approach to the recovery of biological active oligosaccharides from milk using a combination of enzymatic treatment and nanofiltration. Biotechnol. Bioeng 69, 461-467.

In the developed process for the efficient purification of food-grade human milk oligosaccharides from microbial fermentation, nanofiltration is employed in order to concentrate the desired product and also to remove membrane permeable salts.

EP 2 479 263 A1 discloses a method for producing 2'-fucosyllactose using an alpha-1,2-fucosyltransferase from *E. coli.*

WO 2012/092160 A2 discloses nutritional compositions including human milk oligosaccharides that can be administered to individuals including preterm infants, infants, toddlers, and children.

WO 2013/025104 A1 discloses nutritional compositions comprising oligosaccharides and to the uses thereof.

WO 2012/112777 A2 discloses compositions and methods for engineering bacteria to produce fucosylated oligosaccharides, and the use thereof in the prevention or treatment of infection.

Sumiyoshi, W. et al. (Br. J. Nutr., 89(1):61-9) disclose the concentrations of major neutral oligosaccharides in the milk of sixteen Japanese women, collected at 4, 10, 30 and 100 days postpartum.

EP 2 857 410 A1 discloses a process for the purification of 2'-fucosyllactose produced by fermentation, enzymatic synthesis or chemical synthesis or combinations thereof.

WO 2013/185780 A1 discloses a method comprising spray-drying an aqueous solution of an HMO or HMO precursor to remove at least 90 % of the water, and providing an HMO, HMO precursor or HMO blend with a specific glass transition temperature.

CN 101 691 538 A discloses a method for preparing galacto-oligosaccharides, comprising an Aspergillus oryzae fermentation, ceramic membrane ultrafiltration and nanofiltration separation.

Urrutia, P. et al. (J. Agric. Food Chem., 61(5):1081-1087) disclose a catalyzation of the synthesis of galactooligosaccharides (GOS) from lactose by β-galactosidase from *Aspergillus oryzae* (Enzeco).

Starting from this prior art, the technical problem is the provision of a novel process to provide neutral HMOs in high amounts, high purity and excellent yields.

The technical problem is solved by the human milk oligosaccharide (HMO) according to claim 1 and the use of the HMO according to claim 11. The dependent claims display advantageous embodiments.

The invention is set out in the appended set of claims. Any reference hereinafter to the present "disclosure" must be understood as referring to subject matter provided for reference purposes.

The present disclosure provides a process for purification of neutral human milk oligosaccharides (HMO) in a batch manner or in a continuous manner from a fermentation broth obtained by microbial fermentation wherein a purified solution comprising a neutral HMO at a purity of ≥ 80 % is provided. The fermentation broth contains the neutral HMO, biomass, medium components and contaminants. The purity of the neutral HMO in the fermentation broth is <80 %.

During the process the fermentation broth is applied to the following purification steps:
i) Separation of biomass from the fermentation broth,
ii) Cationic ion exchanger treatment for the removal of positively charged material,
iii) Anionic ion exchanger treatment for the removal of negatively charged material,
iv) Nanofiltration step (comprising or consisting of concentration and/or diafiltration of the neutral HMO) and/or electrodialysis step (especially for the removal of salts and other low molecular weight compounds).

Contaminants present in the cell-free fermentation broth are for example other oligosaccharides than the desired neutral HMO, like monovalent and divalent salts, amino acids, polypeptides, proteins, organic acids, nucleic acids, etc. The desired neutral HMO can be obtained at a purity of ≥ 80 % in the purified solution.

The applicant has discovered that with the disclosed purification process, an efficient purification of neutral HMOs from microbial fermentation can be attained, which delivers the HMO at purity suitable for food and feed applications. Furthermore the process is highly cost effective because no chromatographic separation step is needed. Furthermore, it has been discovered that the purity of ≥ 80 % is achieved whether an electrodialysis step or a nanofiltration step is performed in step iv) or if both steps are performed in succession.

One advantage of the process according to the present is that the desired neutral HMOs are obtained free from DNA and proteins from the used recombinant microbial fermentation strain. Especially the implementation of a cationic ion exchanger treatment (step ii) allows the removal of positively charged material like e.g. positively charged proteins. Consequently, the disclosed method provides an HMO which comprises less positively charged contaminant material compared to conventional purification schemes known in the prior art which do not implement a cationic exchanger treatment. It was further discovered that after passing the fermentation broth separated from the biomass (preferably by ultrafiltration) over a cationic ion exchanger (in proton form), the obtained solution was stable and could be stored at room temperature or under cooling for several weeks. Furthermore, the obtained neutral HMO is free of recombinant material, as judged by quantitative PCR with up to 50 amplification cycles. Moreover, the product obtained from the process according to the disclosure is characterized by low amounts or absence of proteins.

Furthermore, the neutral HMO purification according to the disclosure is highly efficient with yet unknown yields of >70 % (optionally > 75%) of the purified HMO (determined from cell free fermentation medium to HMO concentrate).

Thus, a hybrid process is provided comprising the steps of separation of biomass, ion exchanger, and a step of nanofiltration and/or electrodialysis, and preferably further comprising an activated carbon treatment, for the efficient provision of neutral HMOs at high purity free of recombinant genetic material, endotoxins and proteins form fermentation processes using recombinant fermentation strains. With the process according to the disclosure, large amounts of high quality human milk oligosaccharides may be provided in a very convenient and economical way.

The neutral HMO may be purified from a fermentation broth obtained by microbial fermentation using a recombinant microorganism, preferably bacteria or yeast, more preferably a recombinant microorganism grown in a chemically defined medium. Optionally, the biomass separated in step i) is recycled to the microbial fermentation.

In another preferred embodiment of the process according to the disclosure, the purity of the neutral HMO in the fermentation broth is ≤40 %, ≤30 %, ≤20 %, ≤10 % or ≤5 % and/or the purified solution contains the neutral HMO at a purity of ≥85 %, preferably of ≥90 %.

In another preferred embodiment of the process according to the disclosure, the yield of the neutral HMO is >70 % (optionally > 75%) and/or the purified solution is free of DNA, proteins, and/or recombinant genetic material.

In another preferred embodiment of the process according to the disclosure, the neutral HMO is selected from the group consisting of 2'-fucosyllactose 3-fucosyllactose, 2',3-difucosyllactose, lacto-N-triose II, lacto-N-tetraose, lacto-N-neotetraose, lacto-N-fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose and lacto-N-neohexaose.

In another preferred embodiment of the process according to the disclosure, the separation of biomass from the fermentation broth is achieved by
a) ultrafiltration, preferably by separating biomass and materials > 500 kDa, more preferably > 150 kDa; and/or
b) filtration through a cross-flow filter, preferably with a cut-off ≤ 100 kDa, more preferably with a cut-off ≤ 10 kDa, even more preferably a cut-off ≤ 5 kDa;
wherein step a) is preferably implemented before step b).

In another preferred embodiment of the process according to the disclosure, at least one of the purification steps ii) to v) of the disclosed process is repeated at least one time during the process.

In another preferred embodiment of the process according to the disclosure, the fermentation broth is applied at least one time to an activated carbon treatment after at least one of the purification steps i) to iv) for the adsorption of colour giving material and larger oligosaccharides to activated carbon. By applying the fermentation broth to this additional purification step, colour giving material and larger oligosaccharides can be removed from the fermentation broth.

The disclosed process may be characterized in that
a) after at least one of the purification steps i) to iv); or
b) after at least one activated carbon treatment for the adsorption of colour giving material and larger oligosaccharides to activated carbon; or
c) before a concentration step which is implemented after at least one of the purification steps i) to iv);
the solution comprising the neutral human milk oligosaccharide is diafiltered and/or concentrated. Preferably, said solution is diafiltered and/or concentrated with a nanofiltration membrane, more preferably with a nanofiltration membrane having a size exclusion limit of ≤20 Å. Most preferably, the solution is diafiltered until a conductivity of ≤15 mS/cm, preferably ≤10 mS/cm, more preferably ≤5 mS/cm, is reached.

Diafiltration using nanofiltration was found efficient as a pretreatment to remove significant amounts of contaminants prior to an electodialysis treatment of the HMO containing solution. In addition, nanofiltration was also found to be efficient in the removal of low molecular contaminants after an ultrafiltration step, wherein said removal is beneficial for concentrating and demineralizing the HMO solution prior to ion-exchanger treatment. The use of nanofiltration membranes for concentration and diafiltration in the purification of human milk oligosaccharides results in lower energy and processing cost, as well as in improved product quality, due to reduced thermal exposure, leading to reduced Maillard reactions and aldol reactions.

In a step before step i), a glucosidase treatment, preferably a β-glucosidase treatment, may be performed with the fermentation broth, wherein said treatment is preferably performed by
a) adding a microorganism strain capable of expressing one or more glycosidase enzyme(s) which are suitable for the degradation of unwanted intermediates, substrates and/or oligosaccharide side products; and/or
b) using a fermentation strain that expresses one or more glycosidase enzymes(s), preferably by adding an inducer to the fermentation broth and/or by shifting the temperature of the fermentation broth; and/or
c) adding one or more glycosidase(s), preferably at least a β-glucosidase, to the fermentation broth as a crude enzyme or as a purified enzyme.

In another preferred embodiment of the process according to the disclosure, the fermentation broth is concentrated after at least one of the purification steps i) to iv), preferably after purification step iv), using vacuum evaporation or reverse osmosis or nanofiltration (e.g. nanofiltration with a nanofiltration membrane having a size exclusion limit of ≤20 Å)
a) to a concentration of ≥ 100 g/L, preferably ≥ 200 g/L, more preferably ≥ 300 g/L; and/or
b) at a temperature of < 80°C, preferably < 50°C, more preferably 20 °C to 50 °C, even more preferably 30 °C to 45 °C, most preferably 35 °C to 45 °C (specifically relevant for vacuum evaporation or reverse osmosis); and/or
c) at a temperature of < 80 °C, preferably < 50°C, more preferably 4 °C to 40 °C (specifically relevant for nanofiltration).

In another preferred embodiment of the process according to the disclosure, the purified solution is sterile filtered and/or subjected to endotoxin removal, preferably by filtration of the purified solution through a 3 kDa filter.

In another preferred embodiment of the process according to the disclosure, the neutral HMO containing solution is subjected to electrodialysis in order to further remove charged materials such as mono- and divalent salts.

In another preferred embodiment of the process according to the disclosure, the purified solution is concentrated to a concentration of > 1.5 M and cooled to a temperature < 25°, more preferable < 8°C, to obtain crystalline material of the neutral HMO.

In another preferred embodiment of the process according to the disclosure, the purified solution is spray-dried, particularly spray-dried at a concentration of the neutral HMO of 20-60 (w/v), preferably 30-50 (w/v), more preferably 35-45 (w/v), a nozzle temperature of 110-150 °C, preferably 120-140 °C, more preferably 125-135 °C and/or an outlet temperature of 60-80 °C, preferably 65-70 °C.

The present invention provides a neutral human milk oligosaccharide (HMO) that is producible with a process for purifying neutral human milk oligosaccharides from a fermentation broth obtained by microbial fermentation, the fermentation broth comprising a neutral HMO, biomass, medium components and contaminants, wherein the purity of the neutral HMO in the fermentation broth is < 80 %, wherein the fermentation broth is applied to the following purification steps:
i) separation of biomass from the fermentation broth,
ii) cationic ion exchanger treatment for the removal of positively charged material,
iii) anionic ion exchanger treatment for the removal of negatively charged material,
iv) electrodialysis step for the removal of charged materials,

wherein a purified solution comprising the neutral HMO at a purity of ≥ 80% is provided,
wherein the neutral HMO is free of any DNA and free of any proteins,
wherein the neutral HMO is spray-dried,
wherein the neutral HMO is selected from the group consisting of 3'-fucosyllactose, 2',3'-difucosyllactose, lacto-*N*-triose II, lacto-*N*-tetraose, lacto-*N*-neotetraose, lacto-*N*-fucopentaose I, lacto-*N*-neofucopentaose, lacto-*N-*fucopentaose II, lacto-*N*-fucopentaose III, lacto-*N*-fucopentaose V, lacto-*N-neo*fucopentaose V, lacto-*N*-difucohexaose I, lacto-*N*-difucohexaose II, 6'-galactosyllactose, 3'-galactosyllactose, lacto-*N*-hexaose and lacto-*N-*neohexaose.

In the process for providing the inventive neutral HMO, the purity of the neutral HMO in the fermentation broth can be ≤70 %, ≤60 %, ≤50 %, ≤40 % and/or the purified solution can contain the neutral HMO at a purity of ≥ 85 %, preferably of ≥ 90%.

Moreover, in the process for providing the inventive neutral HMO, the separation of biomass from the fermentation broth can be achieved by filtration through a cross-flow filter, preferably with a cut-off ≤ 100 kDa, more preferably with a cut-off ≤ 10 kDa.

Furthermore, in the process for providing the inventive neutral HMO, at least one of the purification steps ii) to iv) can be repeated at least one time during the process.

Besides, in the process for providing the inventive neutral HMO, the fermentation broth can be applied at least one time to an activated carbon treatment after at least one of the purification steps i) to iv) for the adsorption of colour giving material and larger oligosaccharides to activated carbon.

What is more, in the process for providing the inventive neutral HMO, the fermentation broth can be concentrated after at least one of the purification steps i) to iv), preferably after purification step iv), using vacuum evaporation or reverse osmosis, to a concentration of ≥ 100 g/L, preferably ≥ 200 g/L, more preferably ≥ 300 g/L.

In addition, in the process for providing the inventive neutral HMO, the purified solution can be sterile filtered and/or subjected to endotoxin removal, preferably by filtration of the purified solution through a 3 kDa filter.

In the process for providing the inventive neutral HMO, the purified solution can be spray-dried at a concentration of the neutral HMO of 20-60 (w/v), preferably 30-50 (w/v), more preferably 35-45 (w/v), a nozzle temperature of 110-150°C, preferably 120-140°C, more preferably 125-135°C and/or an outlet temperature of 60-80°C, preferably 65-70°C.

Moreover, in the process for providing the inventive neutral HMO, the HMO can have a conductivity of less than 1 mSi/cm at a 300 g/l solution.

Another preferred embodiment is directed to a neutral HMO according to the invention for use in medicine, preferably for use in prophylaxis or therapy of a gastrointestinal disorder.

Furthermore, the present invention includes the use of a neutral HMO according to the invention as additive in food, preferably as additive in human food and/or pet food, more preferably as additive in human baby food.

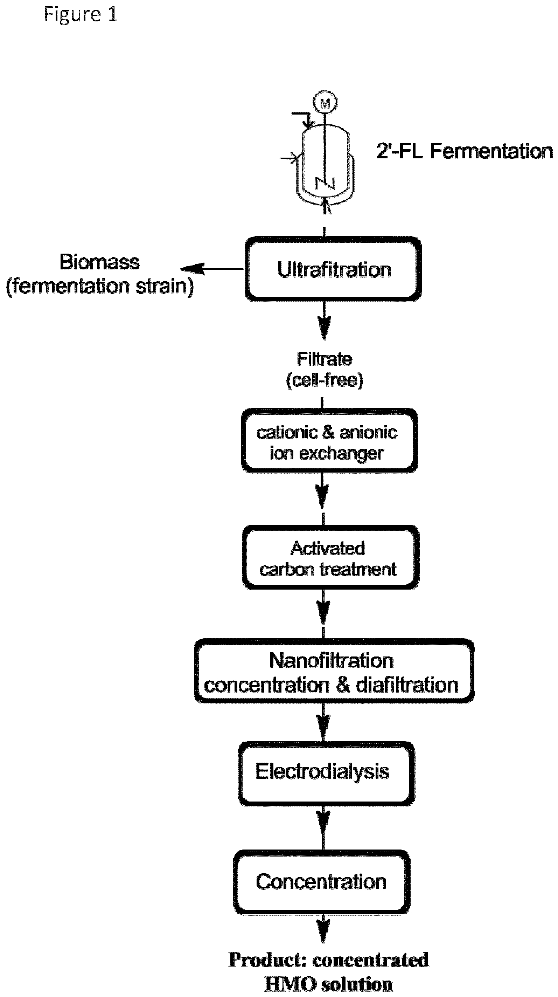

The subject according to the application is intended to be explained in more detail with reference to the subsequent figures and examples without wishing to restrict said subject to the special embodiments. Figures 1 to 3 and Examples 1 and 2 are no embodiments according to the invention, but helpful to understand certain aspects thereof.

Fig. 1 shows a scheme of a preferred embodiment of the process according to the present disclosure for the purification of 2'-fucosyllactose from a fermentation broth comprising the steps: ultrafiltration, cationic and anionic ion exchanger treatment, activated carbon treatment, nanofiltration, electrodialysis and concentration.

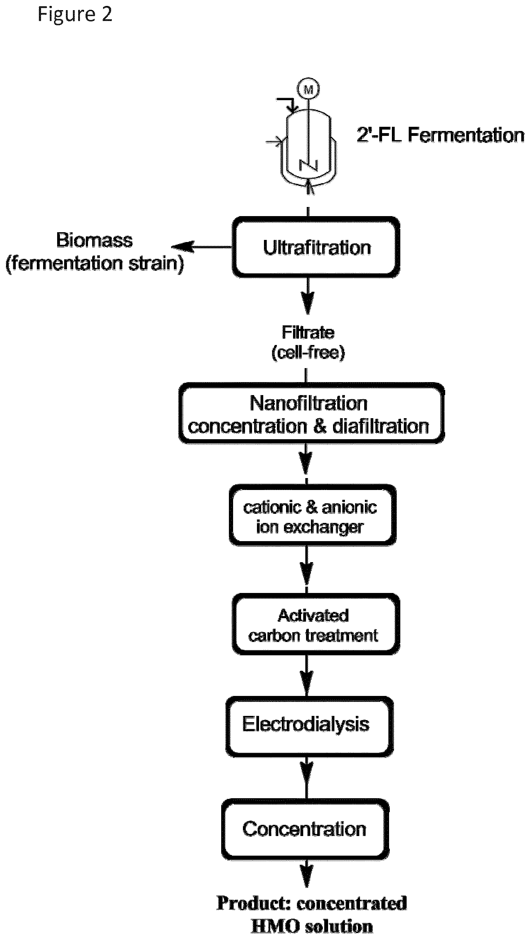

Fig. 2 shows a scheme of another preferred embodiment of the process according to the present disclosure for the purification of 2'-fucosyllactose from a fermentation broth comprising the steps: ultrafiltration, nanofiltration, cationic and anionic ion exchanger treatment, activated carbon treatment, electrodialysis and concentration.

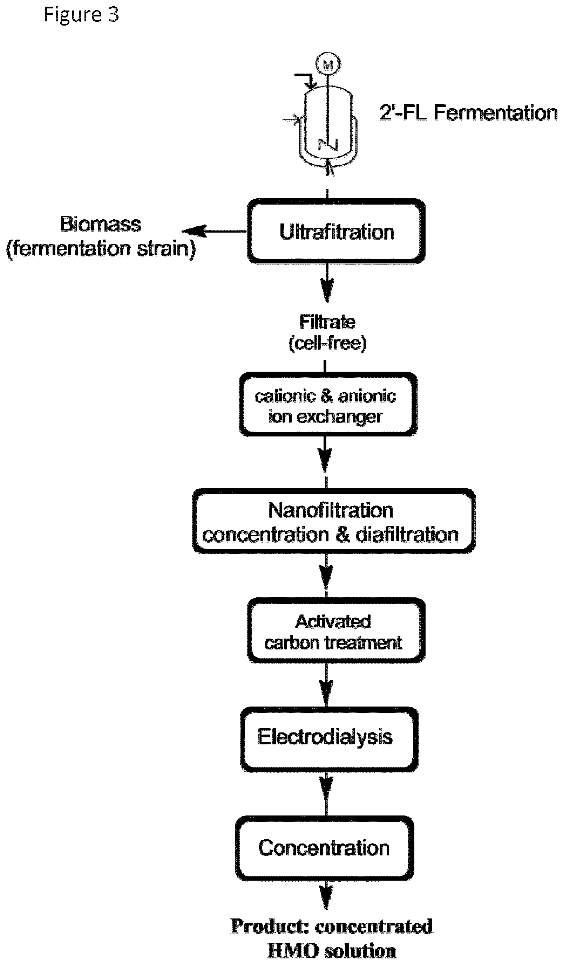

Fig. 3 shows a scheme of another preferred embodiment of the process according to the present disclosure for the purification of 2'-fucosyllactose from a fermentation broth comprising the steps: ultrafiltration, cationic and anionic ion exchanger treatment, nanofiltration, activated carbon treatment, electrodialysis and concentration.

### Example 1: Purification of 2'-fucosyllactose from fermentation using a recombinant microbial production strain I.

A 2'-fucosyllactose feed-batch fermentation employing a recombinant 2'-fucosyllactose synthesizing *E. coli* strain (*E. coli* BL21(DE3) *ΔlacZ*)*,* containing a genomic integration 2'-fuosyltranferase, encoded by the *wbgL* gene (see EP 11 1151 571.4), and having an additional copy of the *E. coli lacY, manB, manC, gmd and fcl* all under the control of a strong constitutive tetracyclin promoter, containing a functional gal operon comprising the genes *galM, galK, galT* and *galE,* was grown in a defined salt medium. The defined salt medium comprised 7 g I⁻¹ NH₄H₃PO₄, 7 g I⁻¹ K₂HPO₄, 2 g I⁻¹ KOH, 0.37g I⁻¹ citric acid, 1 ml I⁻¹ antifoam (Struktol J673, Schill + Seilacher), 1 mM CaCl₂, 4 mM MgSO₄, traceelements and 2% glycerol as carbon source.

Trace elements consisted of 0.101 g I⁻¹ nitrilotriacetic acid, pH 6.5, 0.056 g I⁻¹ ammonium ferric citrate, 0.01 g I⁻¹ MnCl₂ x 4 H₂O, 0.002 g I⁻¹ CoCl₂ x 6 H₂O, 0.001g I⁻¹ CuCl₂ x 2 H₂O, 0.002 g I⁻¹ boric acid, 0.009 g I⁻¹ ZnSO₄ x 7 H₂O, 0.001 g I⁻¹ Na₂MoO₄ x 2 H₂O, 0.002 g I⁻¹ Na₂SeO₃, 0.002 g I⁻¹ NiSO₄ x 6 H₂O.

Glycerol-feed consisted of glycerol 800 g I⁻¹, MgSO₄ 2.64 g I⁻¹ and trace element solution 4 ml I⁻¹. For 2'-fucosyllactose formation, a lactose feed of 216 g I⁻¹ was employed. The pH was controlled by using ammonia solution (25% v/v). Feed batch fermentation was cultured at 30°C under constant aeration and agitation for 90 hours. At 90 hours after the start of the fermentation, most of the added lactose was converted into 2'-fucosyllactose. In order to remove lactose still present in the fermentation supernatant, a second bacterial strain was added to the fermentation vessel 90 hours after the fermentation start.

The added second bacterial strain was genetically identical to the first employed bacteria strain, differing, however, only in the expression of a genome integrated beta-galactosidase. Incubation of the added secondary bacterial strain resulted in the disappearance of the residual lactose within 5 hours. Approximately 10 ml starter culture of the second beta-galactosidase expressing bacterial strain was added per 1I fermentation broth.

The biomass was then separated from the fermentation medium by ultrafiltration, using a cross-flow filter with a cut-off of 10 kDa (Microdyn Nardir).

An approximately 1 m³ cell-free fermentation medium was obtained containing 42 g/l 2'-fucosyllactose.. The cell-free fermentation medium was then passed over a strong cationic ion exchanger (Lewatit S 6368 A (Lanxess) in H⁺ form, size of ion exchanger bed volume was 100 I), in order to remove positive charged contaminants. The obtained solution was then set to pH 7 by the addition of a 2 M sodium hydroxide solution.

The solution was then (without delay) passed over an anionic ion exchanger column (bed volume of ion exachanger was 100 I). The used strong anionic ion exchanger Lewatit S 2568 (Lanxess) was in chloride (Cl⁻) form. The obtained solution was again neutralized to pH 7. The thus obtained solution was then diafiltrated using an Alfa-Laval NF99HF nanofiltration membrane and six volumes of sterile deionized water. The solution was further concentrated using the nanofiltration membrane wherein a 2'-fucosyllactose solution of 200 g/l and a conductivity of 7 mS/cm was obtained.

The concentrated 2'-fucosyllactose solution was then treated with activated carbon in order to remove color giving material such as Maillard reaction products and aldol reaction products. As activated carbon 20 g Norit GAC EN per I concentrated 2'-fucosyllactose solution was used, yielding a significantly decolorized solution.

The thus obtained concentrated 2'-fucosyllactose solution was then electrodialysed to 0.3 mS/cm using a PC-Cell BED 1-3 electrodialysis apparatus (PC-Cell, Heusweiler, Germany) equipped with PC-Cell E200 membrane stack. Said stack contained the following membranes: cation exchange membrane CEM: PC SK and the anion exchange membrane AEM:PcAcid60 having a size exclusion limit of 60 Da. A 0.025 M sulfamic acid (amidosulfonic acid) solution was used as an electrolyte in the ED process.

Then, the obtained solution was then concentrated under vacuum at 40°C to obtain a 45% 2'-fucosyllactose solution. The concentrated solution was then again treated with ion exchangers, Lewatit S 6368 A (Lanxess) in Na⁺ form (bed volume of the used ion exchanger was 10 I) and after neutralization with the anionic ion exchanger Lewatit S 2568 (Lanxess) in Cl⁻ form (bed volume of the employed ion exchanger was 10 I).

The obtained 2'-fucosyllactose solution was then treated with activated carbon (Norit DX1 Ultra). For 1 I of a 45% 2'-fucosyllactose solution 30 g activated carbon were employed.

The solution was then again subjected to electrodialysis until a conductivity of less than 0.3 mSi/cm was obtained.

Subsequently, the solution was subjected to sterile filtration by passing the solution through a 3 kDa filter (Pall Microza ultrafiltration hollow fiber module SEP-2013, Pall Corporation, Dreieich).

Part of the obtained 2'-fucosyllactose solution was then spray dried for analysis.

For NMR spectra recording the spray-dried product was dissolved in hexadeuterodimethyl sulfoxide (DMSO-d₆). For the proton and ¹³C analysis the following characteristic chemical shifts were observed:
**¹H NMR** (500 MHz, DMSO-*d*₆) δ 6.63 (d, *J* = 6.5 Hz, 1H), 6.28 (d, *J* = 4.7 Hz, 1H), 5.21 (d, *J* = 2.4 Hz, 1H), 5.19 (d, *J* = 2.4 Hz, 1H), 5.01 (d, *J* = 2.2, 2H), 4.92 (d, *J =* 5.0 Hz, 1H), 4.89 (dd, *J* = 4.6, 1.3 Hz, 2H), 4.78 (d, *J* = 5.3 Hz, 1H), 4.74 (d, *J =* 5.1 Hz, 1H), 4.63 (m, 6H), 4.53 (t, d, *J* = 5.5, 1H), 4.46 (d, *J* = 5.2 Hz, 1H), 4.44 (d, *J* = 5.0 Hz, 1H), 4.38 - 4.26 (m, 5H), 4.23 (d, *J* = 0.9, 1H), 4.05 (d, *J* = 0.9, 1H), 4.00 (quin, *J* = 3.3, 1H), 3.68 - 3.60 (m, 7H), 3.59 - 3.50 (m, 13H), 3.50 - 3.37 (m, 6H), 3.24 (dt, *J* = 8.8, 2.2 Hz, 1H), 3.14 (m, 2H), 2.96 (td, *J* = 8.4, 4.7 Hz, 1H), 1.04 (d, *J* = 6.1 Hz, 3H), 1.03 (d, *J* = 6.1 Hz, 3H).
**¹³C NMR** (126 MHz, DMSO-d₆) δ 100.99, 100.85, 100.35, 100.25, 96.59, 92.02, 78.13, 77.78, 77.16, 77.01, 75.27 75.05, 74.67, 73.70, 72.33, 71.62, 71.56, 70.91, 69.90, 69.64, 68.75, 68.16 , 66.33, 60.17, 59.82, 59.67, 16.37, 16.36.

Chemicals shifts were found to be consistent with the 2'-fucosyllactose structure.

Using this protocol a 45% 2'-fucosyllactose concentrate with a purity of 94.5 % could be obtained (determined by HPLC analysis). Major contaminants were 3'-fucosyllactose (1.8%), difucosyllactose (2.9%), and lactose (0.3%).

The yield of the purification was approximately 70%.

Importantly, no recombinant material could be determined in 10 g of freeze material using 50 cycles of qPCR. Protein amount of the obtained material was determined as < 50 µg/g freeze dried material by using a nano-Bradford assay (Roth, Karlsruhe Germany). Total amount of ash was determined with 0.19%. Concentration of heavy metals was (arsenic cadmium, lead and mercury) below 0.1 µg/g material. Endotoxin levels were determined to be < 0.005 EU/ml 2'-fucosyllactose concentrate.

### Example 2: Purification of 2'-fucosyllactose from fermentation using a recombinant microbial production strain II.

A 1 m³ microbial fermentation comprising 2'-fucosyllactose at a concentration of 47 g/L was filtered through a cross flow filter with a cut off of 100 kDa (Microdyn Nadir) to obtain a cell free fermentation medium.

As a fermentation medium the following medium was employed: Major medium components: glycerol 30 g/l, NH₄H₂PO₄7 g/l, K₂HPO₄7 g/l, citrate 0.3 g/l, KOH 2 g/l, MgSO₄·7H₂O 2 g/l; trace elements: CaCl₂·6H₂O 20 mg/l, nitrilotriacetic acid 101 mg/l, ammonium ferric citrate 56 mg/l, MnCl₂·4H₂O 9.8 mg/l, CoCl₂·6H₂O 1.6 mg/l, CuCl₂·2H₂O 1 mg/l, H₃BO₃ 1.6 mg/l, ZnSO₄·7H₂O 9 mg/l, Na₂MoO₄·2H₂O 1.2 mg/l, Na₂SeO₃ 1.2 mg/l; feed substances: glycerol and lactose.

The cell free fermentation medium was then passed over a cationic ion exchanger (Lewatit S 6368 A (Lanxess) in H⁺ form (volume of ion exchanger bed was 100 I) in order to remove positive charged contaminants. The obtained solution was then set to pH 7 by the addition of a 2 M sodium hydroxide solution. The solution was then, without delay passed over an anionic ion exchanger column (ion exchanger bed volume used was 100 I) comprising the strong anionic ion exchanger Lewatit S 2568 (Lanxess) in cloride (Cl⁻) form. The obtained solution was again neutralized to pH 7. The so obtained solution was then diafitrated (using 10 volumes of sterile deionized water) and concentrated using a nanofiltration membrane (Alfa-Laval NF99HF) to obtain a 2'-fucosyllactose solution of 200 g/l and a conductivity of approx. 7 mSi/cm.

The concentrated 2'-fucosyllactose solution was then treated with activated carbon, using 20 g Norit GAC EN per I concentrated 2'-fucosyllactose solution. To the filtered 2'-fucosyllactose solution 40 g/l Norit DX1 Ultra activated carbon was added. The solution was then exposed to the activated carbon at 4°C for approximately 18h. After 18h, the activated carbon was removed from the 2'-fucosyllactose solution by filtration.

The solution was then electrodialysed to a conductivity of < 0.3 mS/cm using a PC-Cell BED 1-3 electrodialysis apparatus (PC-Cell, Heusweiler, Germany) equipped with PC-Cell E200 membrane stack. Said stack contained the following membranes: cation exchange membrane CEM: PC SK and the anion exchange membrane AEM:PcAcid60 having a size exclusion limit of 60 Da. A 0.025 M sulfamic acid (amidosulfonic acid) solution was used as an electrolyte in the ED process.

The obtained solution was then concentrated to obtain a 40% 2'-fucosyllactose solution. The obtained 2'-fucosyllactose solution was then passed over a Lewatit S 2568 (Lanxess) Cl⁻ form (bed volume 10I) and treated with activated carbon (Norit DX1 Ultra) at 8°C for 18h. The solution was then subjected to sterile filtration by passing the solution through a 3 kDa filter (Pall Microza ultrafiltration hollow fiber module SEP-2013, Pall Corporation, Dreieich) and spray-dried using a NUBILOSA LTC-GMP spray dryer (NUBILOSA, Konstanz, Germany).

Using this protocol, 2'-fucosyllactose with a purity of 94 % could be obtained (determined by HPLC analysis). Major contaminants were 3'-fucosyllactose (1.8 %), difucosyllactose (3.2 %) and lactose (0.2 %). The yield of the purification was approximately 70 %.

## Claims

1. A neutral human milk oligosaccharide (HMO) producible with a process for purifying neutral human milk oligosaccharides from a fermentation broth obtained by microbial fermentation, the fermentation broth comprising a neutral HMO, biomass, medium components and contaminants, wherein the purity of the neutral HMO in the fermentation broth is < 80 %, wherein the fermentation broth is applied to the following purification steps:
i) separation of biomass from the fermentation broth,
ii) cationic ion exchanger treatment for the removal of positively charged material,
iii) anionic ion exchanger treatment for the removal of negatively charged material,
iv) electrodialysis step for the removal of charged materials,
wherein a purified solution comprising the neutral HMO at a purity of ≥ 80% is provided,
wherein the neutral HMO is free of any DNA and free of any proteins,
wherein the neutral HMO is spray-dried,
wherein the neutral HMO is selected from the group consisting of 3'-fucosyllactose, 2',3'-difucosyllactose, lacto-*N*-triose II, lacto-*N-*tetraose, lacto-*N*-neotetraose, lacto-*N*-fucopentaose I, lacto-*N-neo*fucopentaose, lacto-*N-*fucopentaose II, lacto-*N*-fucopentaose III, lacto-*N*-fucopentaose V, lacto-*N*-neofucopentaose V, lacto-*N-*difucohexaose I, lacto-*N*-difucohexaose II, 6'-galactosyllactose, 3'-galactosyllactose, lacto-*N*-hexaose and lacto-*N*-neohexaose.

2. The neutral HMO according to claim 1, wherein the purity of the neutral HMO in the fermentation broth is ≤70 %, ≤60 %, ≤50 %, ≤40 %and/or the purified solution contains the neutral HMO at a purity of ≥ 85 %, preferably of ≥ 90%.

3. The neutral HMO according to any one of claims 1 to 2, wherein the separation of biomass from the fermentation broth is achieved by filtration through a cross-flow filter, preferably with a cut-off ≤ 100 kDa, more preferably with a cut-off ≤ 10 kDa.

4. The neutral HMO according to any one of claims 1 to 3, wherein at least one of the purification steps ii) to iv) is repeated at least one time during the process.

5. The neutral HMO according to any one of claims 1 to 4, wherein the fermentation broth is applied at least one time to an activated carbon treatment after at least one of the purification steps i) to iv) for the adsorption of colour giving material and larger oligosaccharides to activated carbon.

6. The neutral HMO according to any one of claims 1 to 5, wherein the fermentation broth is concentrated after at least one of the purification steps i) to iv), preferably after purification step iv), using vacuum evaporation or reverse osmosis, to a concentration of ≥ 100 g/L, preferably ≥ 200 g/L, more preferably ≥ 300 g/L.

7. The neutral HMO according to one of claims 1 to 6, wherein the purified solution is sterile filtered and/or subjected to endotoxin removal, preferably by filtration of the purified solution through a 3 kDa filter.

8. The neutral HMO according to one of claims 1 to 7, wherein the purified solution is spray-dried at a concentration of the neutral HMO of 20-60 (w/v), preferably 30-50 (w/v), more preferably 35-45 (w/v), a nozzle temperature of 110-150°C, preferably 120-140°C, more preferably 125-135°C and/or an outlet temperature of 60-80°C, preferably 65-70°C.

9. The neutral HMO according to one of claims 1 to 8, wherein the HMO has a conductivity of less than 1 mSi/cm at a 300 g/l solution.

10. The neutral HMO according to one of claims 1 to 9 for use in medicine, preferably for use in prophylaxis or therapy of a gastrointestinal disorder.

11. Use of an HMO according to any one of claims 1 to 10 as additive in food, preferably as additive in human food and/or pet food, more preferably as additive in human baby food.

## Patentansprüche

1. Neutrales humanes Milch-Oligosaccharid (HMO), das mit einem Verfahren zur Reinigung neutraler humaner Milch-Oligosaccharide aus einer durch mikrobielle Fermentation erhaltenen Fermentationsbrühe herstellbar ist, wobei die Fermentationsbrühe ein neutrales HMO, Biomasse, Mediumkomponenten und Kontaminanten umfasst, wobei die Reinheit des neutralen HMO in der Fermentationsbrühe < 80 % ist, wobei die Fermentationsbrühe den folgenden Reinigungsschritten unterzogen wird:
i) Abtrennung von Biomasse aus der Fermentationsbrühe,
ii) Behandlung mit kationischem Ionenaustauscher zur Entfernung von positiv geladenem Material,
iii) Behandlung mit anionischem Ionenaustauscher zur Entfernung von negativ geladenem Material,
iv) Elektrodialyseschritt zum Entfernen geladener Materialien,
wobei eine gereinigte Lösung, die das neutrale HMO in einer Reinheit von ≥ 80 % umfasst, bereitgestellt wird,
wobei das neutrale HMO frei von jeglicher DNA und frei von jeglichen Proteinen ist,
wobei das neutrale HMO sprühgetrocknet ist,
wobei das neutrale HMO ausgewählt ist aus der Gruppe bestehend aus 3'-Fucosyllactose, 2',3'-Difucosyllactose, Lacto-*N*-triose II, Lacto-*N*-tetraose, Lacto-*N*-*neo*tetraose, Lacto-*N*-fucopentaose I, Lacto-*N*-*neo*fucopentaose, Lacto-*N*-fucopentaose II, Lacto-*N*-fucopentaose III, Lacto-*N-*fucopentaose V, Lacto-*N*-*neo*fucopentaose V, Lacto-*N*-difucohexaose I, Lacto-*N*-difucohexaose II, 6'-Galactosyllactose, 3'-Galactosyllactose, Lacto-*N*-hexaose und Lacto-*N-neo*hexaose.

2. Neutrales HMO nach Anspruch 1, wobei die Reinheit des neutralen HMO in der Fermentationsbrühe ≤ 70 %, ≤ 60 %, ≤ 50 %, ≤40 % ist und/oder die gereinigte Lösung das neutrale HMO in einer Reinheit von ≥ 85 %, vorzugsweise von ≥ 90 % enthält.

3. Neutrales HMO nach einem der Ansprüche 1 bis 2, wobei die Abtrennung von Biomasse aus der Fermentationsbrühe durch Filtration durch einen Querstromfilter, bevorzugt mit einem Cut-off ≤ 100 kDa, bevorzugter mit einem Cut-off ≤ 10 kDa, erreicht wird.

4. Neutrales HMO nach einem der Ansprüche 1 bis 3, wobei mindestens einer der Reinigungsschritte ii) bis iv) mindestens einmal während des Verfahrens wiederholt wird.

5. Neutrales HMO nach einem der Ansprüche 1 bis 4, wobei die Fermentationsbrühe nach mindestens einem der Reinigungsschritte i) bis iv) zur Adsorption von farbgebendem Material und größeren Oligosacchariden an Aktivkohle mindestens einmal einer Aktivkohlebehandlung unterzogen wird.

6. Neutrales HMO nach einem der Ansprüche 1 bis 5, wobei die Fermentationsbrühe nach mindestens einem der Reinigungsschritte i) bis iv), bevorzugt nach Reinigungsschritt iv), unter Verwendung von Vakuumverdampfung oder Umkehrosmose auf eine Konzentration von ≥ 100 g/l, bevorzugt ≥ 200 g/l, bevorzugter ≥ 300 g/l, konzentriert wird.

7. Neutrales HMO nach einem der Ansprüche 1 bis 6, wobei die gereinigte Lösung sterilfiltriert und/oder einer Endotoxinentfernung unterworfen wird, vorzugsweise durch Filtration der gereinigten Lösung durch einen 3-kDa-Filter.

8. Neutrales HMO nach einem der Ansprüche 1 bis 7, wobei die gereinigte Lösung bei einer Konzentration des neutralen HMO von 20-60 (Gew./Vol.), vorzugsweise 30-50 (Gew./Vol.), besonders bevorzugt 35-45 (Gew./Vol.), einer Düsentemperatur von 110-150 °C, vorzugsweise 120-140 °C, besonders bevorzugt 125-135 °C und/oder einer Auslasstemperatur von 60-80 °C, vorzugsweise 65-70 °C sprühgetrocknet wird.

9. Neutrales HMO nach einem der Ansprüche 1 bis 8, wobei das HMO eine Leitfähigkeit von weniger als 1 mSi/cm bei einer 300-g/l-Lösung aufweist.

10. Neutrales HMO nach einem der Ansprüche 1 bis 9 zur Verwendung in der Medizin, vorzugsweise zur Verwendung bei der Prophylaxe oder Therapie einer gastrointestinalen Erkrankung.

11. Verwendung eines HMO nach einem der Ansprüche 1 bis 10 als Zusatzstoff in Nahrung, vorzugsweise als Zusatzstoff in Nahrung für Menschen und/oder Haustiernahrung, besonders bevorzugt als Zusatzstoff in Nahrung für Babys.

## Revendications

1. Oligosaccharides du lait humain (HMO) neutres pouvant être produits par un procédé de purification des oligosaccharides du lait humain neutres à partir d'un bouillon de fermentation obtenu par fermentation microbienne, le bouillon de fermentation comprenant des HMO neutres, de la biomasse, des composants de milieu et des contaminants, dans lesquels la pureté des HMO neutres dans le bouillon de fermentation est < 80 %, dans lesquels le bouillon de fermentation est appliqué aux étapes de purification suivantes :
i) séparation de la biomasse du bouillon de fermentation,
ii) traitement par échangeur d'ions cationiques pour l'élimination de la matière chargée positivement,
iii) traitement par échangeur d'ions anioniques pour l'élimination de la matière chargée négativement,
iv) étape d'électrodialyse pour l'élimination des matières chargées,
dans lesquels une solution purifiée comprenant les HMO neutres à une pureté ≥ 80 % est fournie,
dans lesquels les HMO neutres sont exempts de tout ADN et exempts de toute protéine,
dans lesquels les HMO neutres sont séchés par pulvérisation,
dans lesquels les HMO neutres sont choisis dans le groupe constitué par le 3'-fucosyllactose, le 2',3'-difucosyllactose, le lacto-*N*-triose II, le lacto-*N*-tétraose, le lacto-*N*-neotétraose, le lacto-*N-*fucopentaose I, le lacto-*N*-neofucopentaose, le lacto-*N*-fucopentaose II, le lacto-*N*-fucopentaose III, le lacto-*N*-fucopentaose V, le lacto-*N*-neofucopentaose V, le lacto-*N*-difucohexaose I, le lacto-*N*-difucohexaose II, le 6'-galactosyllose, le 3'-galactosyllose, le lacto-*N*-hexaose et le lacto-*N-*neohexaose.

2. HMO neutres selon la revendication 1, dans lesquels la pureté des HMO neutres dans le bouillon de fermentation est ≤70 %, ≤60 %, ≤50 %, ≤40 % et/ou la solution purifiée contient les HMO neutres à une pureté ≥ 85 %, de préférence ≥ 90 %.

3. HMO neutres selon l'une quelconque des revendications 1 à 2, dans lesquels la séparation de la biomasse du bouillon de fermentation est réalisée par filtration à travers un filtre à écoulement transversal, de préférence avec un seuil ≤ 100 kDa, plus préférablement avec un seuil ≤ 10 kDa.

4. HMO neutres selon l'une quelconque des revendications 1 à 3, dans lesquels au moins l'une des étapes de purification ii) à iv) est répétée au moins une fois au cours du processus.

5. HMO neutres selon l'une quelconque des revendications 1 à 4, dans lesquels le bouillon de fermentation est soumis au moins une fois à un traitement sur charbon actif après au moins l'une des étapes de purification i) à iv) pour l'adsorption des substances responsables de la coloration chromogène et des oligosaccharides de plus grande taille sur le charbon actif.

6. HMO neutres selon l'une quelconque des revendications 1 à 5, dans lesquels le bouillon de fermentation est concentré après au moins l'une des étapes de purification i) à iv), de préférence après l'étape de purification iv), en utilisant l'évaporation sous vide ou l'osmose inverse, à une concentration ≥ 100 g/L, de préférence ≥ 200 g/L, plus préférablement ≥ 300 g/L.

7. HMO neutres selon l'une quelconque des revendications 1 à 6, dans lesquels la solution purifiée est filtrée stérile et/ou soumise à une élimination d'endotoxine, de préférence par filtration de la solution purifiée à travers un filtre de 3 kDa.

8. HMO neutres selon l'une des revendications 1 à 7, dans lesquels la solution purifiée est séchée par pulvérisation à une concentration des HMO neutres de 20 à 60 (w/v), de préférence de 30 à 50 (w/v), plus préférablement de 35 à 45 (w/v), à une température de buse de 110 à 150 °C, de préférence de 120 à 140 °C, plus préférablement de 125 à 135 °C et/ou à une température de sortie de 60 à 80 °C, de préférence de 65 à 70 °C.

9. HMO neutres selon l'une des revendications 1 à 8, dans lesquels les HMO présentent une conductivité inférieure à 1 mSi/cm à une solution à 300 g/l.

10. HMO neutres selon l'une des revendications 1 à 9 destinés à être utilisés en médecine, de préférence destinés à être utilisés en prophylaxie ou en thérapie d'un trouble gastro-intestinal.

11. Utilisation de HMO selon l'une quelconque des revendications 1 à 10 en tant qu'additif dans les aliments, de préférence en tant qu'additif dans les aliments pour humains et/ou les aliments pour animaux domestiques, plus préférablement en tant qu'additif dans les aliments pour bébés humains.
